# EUROPEAN PATENT SPECIFICATION

(11) **EP 1 437 231 B1**
(45) Date of publication and mention of the grant of the patent: **09.01.2008**
(21) Application number: 02775242.7
(22) Date of filing: 27.09.2002
(51) Int. Cl.: B41M 5/30, C07C 39/15

(54) **DEVELOPER FOR THERMAL RECORDING MATERIAL AND THERMAL RECORDING MATERIALS**
ENTWICKLER FÜR WÄRMEAUFZEICHNUNGSMATERIAL UND WÄRMEAUFZEICHNUNGSMATERIALIEN
REVELATEUR POUR SUPPORT DE GRAVURE THERMIQUE ET MATERIAUX DE GRAVURE THERMIQUES

(30) Priority: 27.09.2001 JP 2001298341
(43) Date of publication of application: 14.07.2004
(73) Proprietor: API Corporation, Osaka-shi, Osaka 541-0046 (JP); NIPPON PAPER INDUSTRIES CO., LTD., Kita-ku, Tokyo 114-0002 (JP)
(72) Inventor: SUGA, Mamoru, API CORPORATION, Osaka-shi, Osaka 541-0046 (JP); SUZUKI, Kaori, API CORPORATION, Chikujo-gun, Fukuoka 871-8550 (JP); OMORI, Takashi, NIPPON PAPER INDUSTRIES CO., LTD., Kita-ku, Tokyo 114-0002 (JP); DATE, Takashi, NIPPON PAPER INDUSTRIES CO., LTD., Kita-ku, Tokyo 114-0002 (JP)
(74) Representative: Weber, Thomas
(86) International application number: PCT/JP2002/010015
(87) International publication number: WO 2003/029017

(56) References cited:
- JP-A- 7 061 144
- JP-A- 8 324 116
- JP-A- 10 166 731
- JP-A- 55 067 494
- JP-A- 57 064 594
- JP-A- 59 114 097
- JP-A- 60 222 288
- JP-A- 61 076 386
- JP-A- 61 268 483
- JP-A- 61 270 189
- JP-A- 63 013 779
- JP-A- 2002 326 464
- US-A- 3 937 864
- PATENT ABSTRACTS OF JAPAN vol. 008, no. 234 (M-334), 26 October 1984 (1984-10-26) & JP 59 114096 A (KOJIN KK), 30 June 1984 (1984-06-30)

## Description

The present invention relates to a developer for thermal recording materials and a thermal recording material. More particularly, the present invention relates to a developer for thermal recording materials comprising a condensate or a condensed composition obtained by reacting a substituted phenol and a ketone compound or an aldehyde compound, and a thermal recording material using said developer.

### Background Art

In general, a thermal recording material is obtained by applying a coating liquid onto a support such as paper, plastic film and the like to form a heat-coloring layer, wherein the coating liquid is obtained by grinding and dispersing a basic dye, which is colorless or in a pale color at ambient temperature, and an organic developer, to give respective fine particles, mixing them and adding a binder, a filler, a sensitizer, a lubricant, other additive and the like to the mixture. By heating with a thermal head, a thermal pen, a laser light and the like, it affords a developed color record. The basic principle of such recording method is considered to be the change of the dye to have a color upon chemical contact of an electron donative dye with an organic developer. Such recording method (i.e., thermal recording method) is characterized in that it is free of the need of complicated treatments such as development, fixing and the like, it can record in a short time using a comparatively economical and simple apparatus, is maintenance free, is free of noise during recording, and the obtained color is very clear, as compared to other recording methods conventionally put to practical use, and has been widely used as a recording material for computer output, printer of electronic calculator and the like, recorder for medical measurement, facsimile, automatic ticket vending machine, label fields, copying machine and the like. As multi-purpose and high performance types of these apparatuses have been progressively provided in recent years, a higher-grade thermal recording material has been demanded. For high speed recording and miniaturization of apparatuses, for example, the thermal energy of the thermal head of recording apparatuses tends to become very small, and a thermal recording material to be used therefor is required to show sufficient color density to afford a high density and clear color image even with a very little energy.

To meet such request, various compounds having phenolic hydroxyl group have been proposed as a developer, and disclosed in, for example, JP-B-40-9309, JP-B-43-4160, JP-B-45-14039, JP-B-51-29830, JP-A-56-144193 and the like. Generally, bisphenol compounds, 4-hydroxybenzoic acid ester and the like have been put to practical use alone or in combination of several kinds thereof. However, the conventional materials such as these are associated with problems of, for example, low thermal response, insufficient color density achieved by high speed recording, inconsistent color densities, time-course changes in the density of color image after recording, discoloration of non-image part (background) during preservation, precipitation of white powder on a surface, which is what is called "blooming", degraded re-printability and the like. There has also been disclosed recently a method using a trisphenol compound as a developer or an antifading agent in JP-A-9-278695, JP-A-2001-96926 and the like, but the use of such compound does not lead to a sufficient color density. While JP-A-58-181686 discloses a method using diphenol compound as a developer, the method described in this publication cannot afford sufficient sensitivity and is insufficient in stability of the recorded images, such as heat resistance, moisture resistance, weather resistance and the like. A method to add a sensitizer or a stabilizer along with a phenol developer is also publicly known as a method to solve such problems. However, while the sensitivity becomes generally high, problems occur depending on the kind of a sensitizer (stabilizer) and mixing ratio in that the non-image area is discolored (color of the ground is aggravated) and preservation stability (heat resistance, moisture resistance, weather resistance) of recorded images and non-image part is not improved sufficiently and the like.

JP-A-83-24116 discloses a heat-sensitive recording material containing compound 1-82. This compound is a molecule according to formula (1) of the present invention wherein n = 0, m = 1, R = t-butyl, X = H, and Y = isopropyl. This document further discloses the presence of an electron-donating dye as well as the said compound I-82 in a heat-sensitive layer, said recording material containing wax.

JP-A-10-166731 discloses a heat-sensitive recording material containing the compounds of formula 8. Two of the said compounds correspond to molecules of formula (1) of the present invention wherein n = 0, m = 2, R = methyl, X = H and Y = 2, 4, 4-trimethylpentyl or isopropyl, with methyl being at the p-position of the hydroxyl group of the phenol group.

US-A-3,937,864 discloses a heat-sensitive recording sheet containing the compounds II-4 to II-7. These compounds correspond to molecules of formula (1) of the present invention wherein n = 0, m = 2, R = t-butyl or methyl or n-butyl, X = H or methyl or cyclopentyl, Y = isopropyl, methyl or cyclopentyl, with methyl or n-butyl being at the p-position of the hydroxyl group of the phenol group. This document further discloses the presence of a basic dye as well as the said compounds II-4 to II-7 in the heat-sensitive coating, said coating containing wax.

In view of the above-mentioned situation, an object of the present invention is to provide a developer for thermal recording materials, which is capable of realizing a thermal recording material capable of affording high colored image density and superior in preservation stability (particularly heat resistance and moisture resistance) of recorded images and non-image areas, while satisfying recent requirements for high sensitivity, and a thermal recording material using said developer.

### Disclosure of the Invention

As a result of the intensive studies in an attempt to achieve the above-mentioned object, the present inventors have found that the sensitivity, color density and preservation stability of recorded images and non-image areas of a thermal recording material can be markedly improved by employing a condensate having two cores and comprising a particular substituted phenol and an aldehyde or ketone compound as a developer for the thermal recording material, and that use of a condensed composition further containing a given amount of at least one kind of condensates having 3 to 5 cores along with the condensate having two cores strikingly enhances the improving effect, which resulted in the completion of the present invention.

Accordingly, the present invention relates to a developer for a thermal recording material comprising a condensed composition of condensates represented by the formula (1): 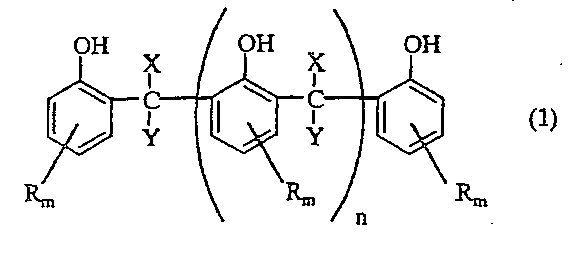 wherein
- R: is a hydrogen atom, a hydroxyl group, a lower alkyl group having 1 to 5 carbon atoms, an alkoxyl group having 1 to 5 carbon atoms, an aryl group or an aralkyl group,
- R: in the number of m may be the same or different and m is an integer of 0-3,
- X and Y: are each a hydrogen atom, an alkyl group or an aryl group, provided that at least one of X and Y is an alkyl group or an aryl group, and
- n: is an integer of 0-3,
characterized in that the condensed composition consists of at least one kind of condensates of the formula (1) wherein n = 1-3 (3-5 core condensate), and 40-98% of the condensate of formula (1) having two cores wherein n = 0. (2) the developer for the thermal recording material of the above-mentioned (1), wherein the condensate has a hydroxyl group, a lower alkyl group having 1 to 5 carbon atoms, an alkoxyl group having 1 to 5 carbon atoms, an aryl group or an aralkyl group at the p-position of a hydroxyl group of a phenol group,
(3) a thermal recording material comprising a heat-coloring layer which comprises the developer of any of the above-mentioned (1) or (2) and a basic dye, and
(4) the thermal recording material of the above-mentioned (3), wherein the heat-coloring layer further comprises one or more kinds of sensitizers.

The present invention is explained in detail in the following.

In the formula (1), n is an integer of 0-3. m is an integer of 0-3, preferably 1-3, more preferably 1. When m is 2 or 3, R in the number of m may be the same or different. When m is 1-3, R is preferably bonded to the m-position or p-position of hydroxyl group of phenol group, more preferably the p-position of hydroxyl group of phenol group. The R in the number of m each shows hydrogen atom, hydroxyl group, alkyl group having 1 to 5 carbon atoms, alkoxyl group having 1 to 5 carbon atoms, aryl group or aralkyl group, preferably alkyl group having 1 to 5 carbon atoms or aralkyl group, particularly preferably alkyl group having 1 to 5 carbon atoms. The above-mentioned alkyl group having 1 to 5 carbon atoms is exemplified by methyl, ethyl, propyl, isopropyl, t-butyl t-amyl and the like, and is preferably methyl and t-butyl. The above-mentioned alkoxyl group having 1 to 5 carbon atoms preferably has 1 to 4 carbon atoms. The above-mentioned alkoxyl group having 1 to 4 carbon atoms is exemplified by methoxy, ethoxy, propoxy, isopropoxy, butoxy, t-butoxy and the like, and is preferably methoxy. The above-mentioned aryl group is exemplified by phenyl, tolyl, naphthyl and the like, and is preferably phenyl. The above-mentioned aralkyl group is exemplified by cumyl, α-methylbenzyl and the like.

In addition, in the formula, X and Y each show a hydrogen atom, an alkyl group or an aryl group, and at least one of X and Y is an alkyl group or an aryl group. It is preferable that either of X and Y be a hydrogen atom, and the other be an alkyl group or an aryl group (particularly preferably the other is an alkyl group). The above-mentioned alkyl group preferably has 1 to 5 carbon atoms, particularly preferably 1 to 4 carbon atoms, and is exemplified by methyl, ethyl, propyl, isopropyl and the like. The above-mentioned aryl group is exemplified by phenyl, tolyl, naphthyl and the like, and is preferably phenyl.

The condensate having two cores, which is represented by the formula (1) (i.e., condensate of the formula wherein n=0), is concretely exemplified by
2,2'-ethylidenebisphenol,
2,2'-ethylidenebis(4-hydroxyphenol),
2,2'-ethylidenebis(5-hydroxyphenol),
2,2'-ethylidenebis(4-methylphenol),
2,2'-ethylidenebis(5-methylphenol),
2,2'-ethylidenebis(4-ethylphenol),
2,2'-ethylidenebis(5-ethylphenol),
2,2'-ethylidenebis(4-propylphenol),
2,2'-ethylidenebis(4-isopropylphenol),
2,2'-ethylidenebis(5-isopropylphenol),
2,2'-ethylidenebis(4-t-butylphenol),
2,2'-ethylidenebis(5-t-butylphenol),
2,2'-ethylidenebis(4-t-amylphenol),
2,2'-ethylidenebis(4-methoxyphenol),
2,2'-ethylidenebis(5-methoxyphenol),
2,2'-ethylidenebis(4-phenylphenol),
2,2'-ethylidenebis(5-phenylphenol),
2,2'-ethylidenebis(4-cumylphenol),
2,2'-ethylidenebis[4-(α-methylbenzyl)phenol],
2,2'-butylidene bisphenol,
2,2'-butylidenebis(4-hydroxyphenol),
2,2'-butylidenebis(5-hydroxyphenol),
2,2'-butylidenebis(4-methylphenol),
2,2'-butylidenebis(5-methylphenol),
2,2'-butylidenebis(4-ethylphenol),
2,2'-butylidenebis(5-ethylphenol),
2,2'-butylidenebis(4-propylphenol),
2,2'-butylidenebis(4-isopropylphenol),
2,2'-butylidenebis(5-isopropylphenol),
2,2'-butylidenebis(4-t-butylphenol),
2,2'-butylidenebis(5-t-butylphenol),
2,2'-butylidenebis(4-t-amylphenol),
2,2'-butylidenebis(4-methoxyphenol),
2,2'-butylidenebis(5-methoxyphenol),
2,2'-butylidenebis(4-phenylphenol),
2,2'-butylidenebis(5-phenylphenol),
2,2'-butylidenebis(4-cumylphenol),
2,2'-butylidenebis[4-(α-methylbenzyl)phenol],
2,2'-(phenylmethylene)bisphenol,
2,2'-(phenylmethylene)bis(4-hydroxyphenol),
2,2'-(phenylmethylene)bis(5-hydroxyphenol),
2,2'-(phenylmethylene)bis(4-methylphenol),
2,2'-(phenylmethylene)bis(5-methylphenol),
2,2'-(phenylmethylene)bis(4-ethylphenol),
2,2'-(phenylmethylene)bis(5-ethylphenol),
2,2'-(phenylmethylene)bis(4-propylphenol),
2,2'-(phenylmethylene)bis(4-isopropylphenol),
2,2'-(phenylmethylene)bis(5-isopropylphenol),
2,2'-(phenylmethylene)bis(4-t-butylphenol),
2,2'-(phenylmethylene)bis(5-t-butylphenol),
2,2'-(phenylmethylene)bis(4-t-amylphenol),
2,2'-(phenylmethylene)bis(4-methoxyphenol),
2,2'-(phenylmethylene)bis(5-methoxyphenol),
2,2'-(phenylmethylene)bis(4-phenylphenol),
2,2'-(phenylmethylene)bis(5-phenylphenol),
2,2'-(phenylmethylene)bis(4-cumylphenol),
2,2[4-(α-methylbenzyl)'-(phenylmethylene)bisphenol]

Of these, 2,2'-ethylidenebis(4-methylphenol), 2,2'-ethylidenebis(t-butylphenol), 2,2'-ethylidenebis(4-cumylphenol), 2,2'-butylidenebis(4-methylphenol), 2,2'-butylidenebis(t-butylphenol) and 2,2'-butylidenebis (4-cumylphenol) are preferable.

Examples of the condensates having 3 to 5 cores, which are represented by the formula (1) (i.e., condensates of the formula wherein n=1-3), include compounds corresponding to the compounds exemplified for the aforementioned condensate having two cores.

The developer for thermal recording material of the present invention (hereinafter also simply referred to as "developer") comprises a condensed composition containing, of the condensates represented by the formula (1), a condensate having two cores, or mainly a condensate having two cores and, further, at least one kind of condensates having 3 to 5 cores. By the "at least one kind of condensates having 3 to 5 cores" in the condensed composition is meant solely a condensate having three cores, two kinds of a condensate having three cores and a condensate having four cores or three kinds of a condensate having three cores, a condensate having four cores and a condensate having five cores. By the "mainly a condensate having two cores" is meant that, of the condensates constituting the condensation composition, the ratio of the condensate having two cores is the highest.

The developer of the present invention is a condensation composition, because it achieves a higher color density and finer preservation stability of the recorded images in comparison with a developer consisting of a condensate having two cores. In addition, the condensed composition comprises the condensate having two cores in a proportion of 40-98% (the rest is at least one kind of condensates having 3 to 5 cores). A content of the condensate having two cores of less than 40% (content of at least one kind of condensates having 3 to 5 cores exceeding 60%) and a content of the condensate having two cores exceeding 98% (content of at least one kind of condensates having 3 to 5 cores of less than 2%) make it difficult to achieve such noticeable improving effect. A more preferable composition of the condensed composition has a content of the condensate having two cores of 50-98% (content of at least one kind of condensates having 3 to 5 cores of 2-50%) and a particularly preferable composition has a content of the condensate having two cores of 60-95% (content of at least one kind of condensates having 3 to 5 cores of 5-40%).

Note that "%" in the present specification means "area %" in the results of the analysis by high performance liquid chromatography.

The developer for thermal recording material of the present invention can be easily produced by a known synthetic method comprising, for example, reacting substituted phenol with a ketone compound or a formaldehyde compound in the presence of an acid catalyst (e.g., hydrochloric acid, p-toluenesulfonic acid and the like), and the like. The reaction is carried out in a suitable organic solvent inert to the reaction, which can dissolve the starting material and reaction product, such as water, methanol, ethanol, n-propyl alcohol, isopropyl alcohol, acetonitrile, toluene, chloroform, diethyl ether, N,N-dimethylacetamide, benzene, chlorobenzene, dichlorobenzene, diethyl ketone, ethyl methyl ketone, acetone, tetrahydrofuran and the like, at a reaction temperature of 0-150°C for several hours to several dozen hours. After the reaction, by removing unreacted substituted phenol by distillation, an object condensate or condensed composition can be obtained in a high yield. In addition, by recrystallizing the condensate or condensed composition obtained in this way in a suitable solvent, a condensate or a condensed composition with a high purity can be also obtained.

Specific examples of the above-mentioned substituted phenols include phenol, catechol, resorcinol, hydroquinone, p-cresol, m-cresol, o-cresol, p-ethylphenol, m-ethylphenol, o-ethylphenol, p-propylphenol, o-propylphenol, p-isopropylphenol, m-isopropylphenol, o-isopropylphenol, p-t-butylphenol, m-t-butylphenol, o-t-butylphenol, p-t-amylphenol, p-methoxyphenol, m-methoxyphenol, o-methoxyphenol, p-phenylphenol, m-phenylphenol, o-phenylphenol, p-cumylphenol, p-(α-methylbenzyl)phenol, o-(α-methylbenzyl)phenol and the like. p-Substituted phenols having at least one unsubstituted o-position are preferable, and of these, p-cresol, p-ethylphenol and p-t-butylphenol are particularly preferable, and p-cresol and p-t-butylphenol are particularly preferable.

Concrete examples of ketone compound and aldehyde compound include, for example, dimethylketone, diethyl ketone, ethyl methyl ketone, methyl isobutyl ketone, benzaldehyde and the like.

The thermal recording material of the present invention comprises a heat-coloring layer containing the developer of the present invention explained above and a basic dye and is usually constituted by forming the heat-coloring layer on a support.

In the thermal recording material of the present invention, the basic dye to be contained in the heat-coloring layer may be any colorless to pale color basic dye known and used in the field of thermal recording material and is not particularly limited. It is preferably a leucodye such as triphenylmethane, fluoran, fluorene, divinyl leucodyes and the like, particularly preferably fluoran leucodye, and most preferably anilinofluoran leucodye. The basic dye may be used alone or in combination of two or more kinds thereof.

Specific examples of the basic dye are shown in the following.
<triphenylmethane leucodye>
3,3-bis(p-dimethylaminophenyl)-6-dimethylaminophthalide
3,3-bis(p-dimethylaminophenyl)phthalide
<fluoran leucodye>
3-diethylamino-6-methylfluoran
3-diethylamino-6-methyl-7-anilinofluoran
3-diethylamino-6-methyl-7-(o,p-dimethylanilino)fluoran
3-diethylamino-6-methyl-7-chlorofluoran
3-diethylamino-6-methyl-7-(m-trifluoromethylanilino)fluoran
3-diethylamino-6-methyl-7-(o-chloroanilino)fluoran
3-diethylamino-6-methyl-7-(p-chloroanilino)fluoran
3-diethylamino-6-methyl-7-(o-fluoroanilino)fluoran
3-diethylamino-6-methyl-7-(m-methylanilino)fluoran
3-diethylamino-6-chloro-7-methylfluoran
3-diethylamino-6-chloro-7-anilinofluoran
3-diethylamino-6-chloro-7-p-methylanilinofluoran
3-diethylamino-7-methylfluoran
3-diethylamino-7-chlorofluoran
3-diethylamino-7-(m-trifluoromethylanilino)fluoran
3-diethylamino-7-(o-chloroanilino)fluoran
3-diethylamino-7-(p-chloroanilino)fluoran
3-diethylamino-7-(o-fluoroanilino)fluoran
3-diethylamino-benzo[a]fluoran
3-diethylamino-benzo[c]fluoran
3-dibutylamino-6-methylfluoran
3-dibutylamino-6-methyl-7-anilinofluoran
3-dibutylamino-6-methyl-7-(o,p-dimethylanilino)fluoran
3-dibutylamino-6-methyl-7-(o-chloroanilino)fluoran
3-dibutylamino-6-methyl-7-(p-chloroanilino)fluoran
3-dibutylamino-6-methyl-7-(o-fluoroanilino)fluoran
3-dibutylamino-6-methyl-7-(m-trifluoromethylanilino)fluoran
3-dibutylamino-6-methyl-7-chlorofluoran
3-dibutylamino-6-chloro-7-anilinofluoran
3-dibutylamino-6-methyl-7-p-methylanilinofluoran
3-dibutylamino-7-(o-chloroanilino)fluoran
3-dibutylamino-7-(o-fluoroanilino)fluoran
3-di-n-pentylamino-6-methyl-7-anilinofluoran
3-di-n-pentylamino-6-methyl-7-(p-chloroanilino)fluoran
3-di-n-pentylamino-7-(m-trifluoromethylanilino)fluoran
3-di-n-pentylamino-6-chloro-7-anilinofluoran
3-di-n-pentylamino-7-(p-chloroanilino)fluoran
3-(N-ethyl-N-cyclohexylamino)-6-methyl-7-anilinofluoran
3-(N-ethyl-p-toluidino)-6-methyl-7-anilinofluoran
3-(N-ethyl-N-isoamylamino)-6-methyl-7-anilinofluoran
3-(N-ethyl-N-isoamylamino)-6-chloro-7-anilinofluoran
3-(N-ethyl-N-tetrahydrofrufurylamino)-6-methyl-7-anilinofluoran
3-(N-ethyl-N-isobutylamino)-6-methyl-7-anilinofluoran
<fluorene leucodye>
3,6,6'-tris(dimethylamino)spiro[fluorene-9,3'-phthalide]
3,6,6'-tris(diethylamino)spiro[fluorene-9,3'-phthalide]
<divinyl leucodye>
3,3-bis[2-(p-dimethylaminophenyl)-2-(p-methoxyphenyl)ethenyl]-4,5,6,7-tetrabromophthalide
3,3-bis[2-(p-dimethylaminophenyl)-2-(p-methoxyphenyl)ethenyl]-4,5,6,7-tetrachlorophthalide
3,3-bis[1,1-bis(4-pyrrolidinophenyl)ethylen-2-yl]-4,5,6,7-tetrabromophthalide
3,3-bis[1-(4-methoxyphenyl)-1-(4-pyrrolidinophenyl)ethylen-2-yl]-4,5,6,7-tetrachlorophthalide
<other basic dyes>
3-(4-diethylamino-2-ethoxyphenyl)-3-(1-ethyl-2-methylindol-3-yl)-4-azaphthalide
3-(4-diethylamino-2-ethoxyphenyl)-3-(1-octyl-2-methylindol-3-yl)-4-azaphthalide
3-(4-cyclohexylethylamino-2-methoxyphenyl)-3-(1-ethyl-2-methylindol-3-yl)-4-azaphthalide
3,3-bis(1-ethyl-2-methylindol-3-yl)phthalide
3,6-bis(diethylamino)fluoran-γ-(3'-nitro)anilinolactam
3,6-bis(diethylamino)fluoran-γ-(4'-nitro)anilinolactam
1,1-bis[2',2',2",2"-tetrakis-(p-dimethylaminophenyl)ethenyl]-2,2-dinitrile ethane
1,1-bis[2',2',2",2"-tetrakis-(p-dimethylaminophenyl)ethenyl]-2-β-naphthoylethane
1,1-bis[2',2',2",2"-tetrakis-(p-dimethylaminophenyl)ethenyl]-2,2-diacetylethane
bis[2,2,2',2'-tetrakis-(p-dimethylaminophenyl)ethenyl]-methylmalonic acid dimethyl ester.

The heat-coloring layer of the thermal recording material of the present invention preferably contains a sensitizer as well as the developer of the present invention and a basic dye. As such sensitizers, sensitizers known in the thermal recording material field may be used without limitation. For example, stearic acid amide, palmitic acid amide, methoxycarbonyl-N-stearic acid benzamide, N-benzoylstearic acid amide, N-eicosanoic acid amide, ethylenebisstearic acid amide, behenic acid amide, methylenebisstearic acid amide, methylolamide, N-methylol stearic acid amide, dibenzyl terephthalate, dimethyl terephthalate, dioctyl terephthalate, benzyl p-benzyloxybenzoate, phenyl 1-hydroxy-2-naphthoate, dibenzyl oxalate, di-p-methylbenzyl oxalate, oxalic acid-di-p-chlorobenzyl, 2-naphthylbenzyl ether, p-benzyl biphenyl, 4-biphenyl-p-tolyl ether, di(p-methoxyphenoxyethyl) ether, 1,2-di(3-methylphenoxy)ethane, 1,2-di(4-methylphenoxy)ethane, 1,2-di(4-methoxyphenoxy)ethane, 1,2-di(4-chlorophenoxy)ethane, 1,2-diphenoxyethane, 1-(4-methoxyphenoxy)-2-(2-methylphenoxy)ethane, p-methylthiophenylbenzyl ether, 1,4-di(phenylthio)butane, p-acetotoluidide, p-acetophenetidide, N-acetoacetyl-p-toluidine, di(β-biphenylethoxy)benzene, p-di(vinyloxyethoxy)benzene, 1-isopropylphenyl-2-phenylethane, 1,2-bis(phenoxymethyl)benzene, p-toluenesulfonamide, o-toluenesulfonamide, di-p-tolyl carbonate, phenyl-α-naphthyl carbonate, diphenylsulfone and the like can be mentioned. Of these, benzyl p-benzyloxybenzoate, stearic acid amide, ethylenebisstearic acid amide, di-p-methylbenzyl oxalate, dip-chlorobenzyl oxalate, 2-naphthylbenzyl ether, p-benzylbiphenyl, 4-biphenyl-p-tolyl ether, 1,2-di(3-methylphenoxy)ethane, 1,2-bis(phenoxymethyl)benzene and diphenylsulfone are preferable, and benzyl p-benzyloxybenzoate, di-p-methylbenzyl oxalate, 2-naphthylbenzyl ether, p-benzylbiphenyl, 4-biphenyl-p-tolyl ether, 1,2-di(3-methylphenoxy)ethane, 1,2-bis(phenoxymethyl)benzene and diphenylsulfone are particularly preferable. One or more kinds of these sensitizers can be used.

The heat-coloring layer of the thermal recording material of the present invention may contain a conventionally known organic developer to the extent that the effect of the present invention is not impaired. Examples of such organic developer include 4-hydroxybenzoic acid esters, 4-hydroxyphthalic acid diesters, phthalic acid monoesters, bis(hydroxyphenyl)sulfides, 4-hydroxyphenylarylsulfones (e.g., 4-(4-propoxy-benzenesulfonyl)phenol, 4-(4-isopropoxybenzenesulfonyl)phenol and the like), 4-hydroxyphenylarylsulfonates, 1,3-di[2-(hydroxyphenyl)-2-propyl]benzenes, 4-hydroxybenzoyloxybenzoic acid esters, bisphenolsulfones and the like. One or more kinds of these organic developers can be used.

Where necessary, inorganic or organic fillers (fillers) such as silica, calcium carbonate, kaoline, calcined kaolin, diatomite, talc, titanium oxide, zinc oxide, aluminum hydroxide, polystyrene resin, urea - formalin resin, styrene - methacrylic acid copolymer, styrene - butadiene copolymer, hollow plastic pigment and the like; stabilizers such as p-nitrobenzoic acid metal salt (Ca, Zn), benzyl monophthalate metal salt (Ca, Zn) and the like; surface lubricants such as fatty acid metal salt and the like; lubricants such as wax and the like; benzophenone or triazole ultraviolet absorbers; waterproof agents such as glyoxal resistance; dispersing agents; antifoaming agents and the like can be added to the heat-coloring layer.

In the present invention, the developer of the present invention, a basic dye, a sensitizer and other additives, which are added as necessary, are dispersed in a solution of a binder or an emulsion of a binder or a dispersion containing a binder in paste therein to give a coating liquid, and the resulting liquid is applied to a support and dried to form a heat-coloring layer. It is preferable to prepare, for each material, a dispersion liquid containing the material and a binder, and a formulating (mixing) the dispersion for each material to ultimately give a coating liquid. In addition, it is also preferable to process, during the preparation process of the coating liquid, the developer, basic dye, sensitizer and other necessary additives using a grinder such as ball mill, Attritor, sand grinder and the like or a suitable emulsification device to achieve a fine particle size of not more than several microns (specifically, average particle size of not more than 3.0 micrometers, preferably 0.5-1.0 micrometers). The average particle size here is a measured value by a Mastersizer.

Examples of the binder to be used in the invention include completely saponified polyvinyl alcohol having a polymerization degree of 200-1900, partially saponified polyvinyl alcohol, carboxy denatured polyvinyl alcohol, amide denatured polyvinyl alcohol, sulfonic acid denatured polyvinyl alcohol, butyral denatured polyvinyl alcohol, other denatured polyvinyl alcohols, hydroxyethyl cellulose, methyl cellulose, carboxymethyl cellulose, styrene-maleic anhydride copolymer, styrene-butadiene copolymer and cellulose derivatives such as ethyl cellulose and acetyl cellulose, polyvinyl chloride, polyvinyl acetate, polyacrylamide, polyacrylic acid ester, polyvinylbutyral polystyrol and copolymers thereof, polyamide resin, silicone resin, petroleum resin, terpene resin, ketone resin, cumarone resin and the like. These polymers can be used alone or in combination of two or more kinds thereof according to the requested quality. They may be dissolved in a solvent such as water, alcohol, ketone, ester, hydrocarbon and the like, or emulsified or dispersed in paste in water or other medium and used.

In the present invention, the amounts of use of each material such as developer, basic dye, sensitizer, binder, filler and the like to be used for the heat-coloring layer are not particularly limited. Generally, 1-8 parts by weight (preferably 1-4 parts by weight) of a developer, 1-8 parts by weight (preferably 1-4 parts by weight) of a sensitizer and 1-20 parts by weight (preferably 1-10 parts by weight) of a filler per 1 part by weight of a basic dye are preferable. A binder is added suitably in an amount of about 10-25 wt% of the entire solid content.

As a support of a heat-coloring layer, paper, recycled paper, synthetic paper, plastic film, foamed plastic film, nonwoven fabric, metal foil and the like can be used, and a composite sheet combining these can be also used.

The amount of presence (attached amount of solid) of the heat-coloring layer on a support is preferably 1-10 g/m², particularly preferably 2-8 g/m², per one surface of the support.

In the thermal recording material of the present invention, moreover, preservability can be enhanced by forming an overcoating layer made of a polymer containing an organic loading material, and the like on the heat-coloring layer. Moreover, preservability and sensitivity can be enhanced by forming an undercoating layer containing an organic and/or an inorganic loading material under the heat-coloring layer.

### Examples

The present invention is explained in detail by referring to Examples and Comparative Examples, which are not to be construed as limitative. In the explanation, "part" means "part by weight". In addition, the composition of condensed composition containing 80% of 2,2'-ethylidenebis(4-methylphenol) and condensed composition containing 70% of 2,2'-ethylidenebis(4-t-butylphenol) are as described below.
Condensation composition containing 80% of 2,2'-ethylidenebis(4-methylphenol)
2,2'-ethylidenebis(4-methylphenol):2,6-bis[1-(2-hydroxy-5-methylphenyl)ethyl]-4-methylphenol:2,2'-ethylidenebis[6-[1-(2-hydroxy-5-methylphenyl)ethyl]-4-methylphenol]=80:9:11
Condensation composition containing 70% of 2,2'-ethylidenebis(4-t-butylphenol)
2,2'-ethylidenebis(4-t-butylphenol):2,6-bis[1-(2-hydroxy-5-t-butylphenyl)ethyl]-4-t-butylphenol:2,2'-ethylidenebis[6-[1-(2-hydroxy-5-t-butylphenyl)ethyl]-4-t-butylphenol]:2,6-bis[1-[2-hydroxy-3-[1-(2-hydroxy-5-t-butylphenyl)ethyl]-5-t-butylphenyl]ethyl]-4-t-butylphenol=70:16:12:2

### [Example 1] (comparative)

| Liquid A (developer dispersion) | |
|---|---|
| 2,2'-ethylidenebis(4-methylphenol) | 6.0 parts |
| 10% aqueous polyvinyl alcohol solution | 18.8 parts |
| water | 11.2 parts |

| Liquid B (sensitizer dispersion) | |
|---|---|
| diphenylsulfone | 4.0 parts |
| 10% aqueous polyvinyl alcohol solution | 12.5 parts |
| water | 7.5 parts |

| Liquid C (dye dispersion) | |
|---|---|
| 3-(N-ethyl-N-isoamylamino)-6-methyl-7-anilinofluoran | 2.0 parts |
| 10% aqueous polyvinyl alcohol solution | 4.6 parts |
| water | 2.6 parts |

The above-mentioned Liquid A, Liquid B and Liquid C were subjected to a grinding treatment by a sand grinder until the developer, sensitizer and dye each had an average particle size of 1.0 micron, and the Liquid A, Liquid B and Liquid C after the treatment were mixed at the following ratio to give a coating liquid.

| | |
|---|---|
| Liquid A (developer dispersion) | 36.0 parts |
| Liquid B (sensitizer dispersion) | 24.0 parts |
| Liquid C (dye dispersion) | 9.2 parts |
| Kaolin clay (50% dispersion) | 12.0 parts |

The above-mentioned coating liquid was applied to one side of a basic paper having a basic weight of 50 g/m² in a coating amount of 6.0 g/m² and dried at room temperature for 24 hr. This sheet was treated with a supercalender to smoothness of 500-600 sec to give a thermal recording material. The coated amount here means the amount of the solid adhered to the support after drying.

### [Example 2] (comparative)

In the same manner as in Example 1 except that 2-benzyloxynaphthalene was used instead of diphenylsulfone used for Liquid B (sensitizer dispersion) in Example 1, a thermal recording material was prepared.

### [Example 3]

In the same manner as in Example 1 except that a condensed composition containing 80% of 2,2'-ethylidenebis(4-methylphenol) was used instead of 2,2'-ethylidenebis(4-methylphenol) used for Liquid A (developer dispersion) in Example 1, a thermal recording material was prepared.

### [Example 4]

In the same manner as in Example 1 except that a condensed composition containing 80% of 2,2'-ethylidenebis(4-methylphenol) was used instead of 2,2'-ethylidenebis(4-methylphenol) used for Liquid A (developer dispersion) in Example 1 and 1,2-bis(phenoxymethyl)benzene was used instead of diphenylsulfone used for Liquid B (sensitizer dispersion), a thermal recording material was prepared.

### [Example 5]

In the same manner as in Example 1 except that a condensed composition containing 70% of 2,2'-ethylidenebis(4-t-butylphenol) was used instead of 2,2'-ethylidene (4-methylphenol) used for Liquid A (developer dispersion) in Example 1, a thermal recording material was prepared.

### [Example 6]

In the same manner as in Example 1 except that a condensed composition containing 70% of 2,2'-ethylidenebis(4-t-butylphenol) was used instead of 2,2'-ethylidenebis(4-methylphenol) used for Liquid A (developer dispersion) in Example 1 and ethylene glycol diphenyl ether was used instead of diphenylsulfone used for Liquid B (sensitizer dispersion), a thermal recording material was prepared.

### [Example 7] (comparative)

In the same manner as in Example 1 except that 2,2'-ethylidenebis(4-cumylphenol) was used instead of 2,2'-ethylidenebis(4-methylphenol) used for Liquid A (developer dispersion liquid) in Example 1, a thermal recording material was prepared.

### [Example 8] (comparative)

In the same manner as in Example 1 except that 2,2'-ethylidenebis(4-cumylphenol) was used instead of 2,2'-ethylidenebis(4-methylphenol) used for Liquid A (developer dispersion) in Example 1 and 1,2-bis(3-methylphenoxy)ethane was used instead of diphenylsulfone used for Liquid B (sensitizer dispersion), a thermal recording material was prepared.

### [Example 9] (comparative)

In the same manner as in Example 1 except that 2,2'-butylidenebis(4-methylphenol) was used instead of 2,2'-ethylidenebis(4-methylphenol) used for Liquid A (developer dispersion) in Example 1, a thermal recording material was prepared.

### [Example 10] (comparative)

In the same manner as in Example 1 except that 2,2'-butylidenebis(4-methylphenol) was used instead of 2,2'-ethylidenebis(4-methylphenol) used for Liquid A (developer dispersion) and 4-biphenyl-p-tolyl ether was used instead of diphenylsulfone used for Liquid B (sensitizer dispersion) in Example 1, a thermal recording material was prepared.

### [Example 11] (comparative)

In the same manner as in Example 1 except that 2,2'-butylidenebis(4-t-butylphenol) was used instead of 2,2'-ethylidenebis(4-methylphenol) used for Liquid A (developer dispersion) in Example 1, a thermal recording material was prepared.

### [Example 12] (comparative)

In the same manner as in Example 1 except that 2,2'-butylidenebis(4-t-butylphenol) was used instead of 2,2'-ethylidenebis(4-methylphenol) used for Liquid A (developer dispersion) and 4-biphenyl-p-tolyl ether was used instead of diphenylsulfone used for Liquid B (sensitizer dispersion) in Example 1, a thermal recording material was prepared.

### [Comparative Example 1]

In the same manner as in Example 1 except that phenol novolac resin (Phenolite TD2090) manufactured by DAINIPPON INK AND CHEMICALS, INCORPORATED was used instead of 2,2'-ethylidenebis(4-methylphenol) of Liquid A (developer dispersion) in Example 1 and diphenylsulfone of Liquid B (sensitizer dispersion) was not used, a thermal recording material was prepared.

### [Comparative Example 2]

In the same manner as in Example 1 except that phenol novolac resin (Phenolite TD2090) manufactured by DAINIPPON INK AND CHEMICALS, INCORPORATED was used instead of 2,2'-ethylidenebis(4-methylphenol) in Liquid A (developer dispersion) of Example 1, a thermal recording material was prepared.

The thermal recording materials produced in the above Examples 1-12 and Comparative Examples 1 and 2 were tested for the following quality and performance. The results are shown in Tables 1 and 2. In the Tables, the numerals in the upper line show the density of the recorded area and those in the lower line show the density of the non-image area.

### [Thermal recordability test (dynamic color density)]

The prepared thermal recording materials were subjected to printing using TH-PMD manufactured by Ohkura Electric Co., Ltd. (thermal printer, equipped with a thermal head manufactured by Kyocera Corporation) at an impression energy of 0.38 mj/dot. The image density of the recorded area was measured with the Macbeth densitometer (RD-914, using Amber Filter).

### [Heat resistance test]

The thermal recording materials printed on in the thermal recordability test were stood for 24 hr in a high temperature dry environment at a test temperature of 60°C, and the image densities of the recorded area and the non-image area were measured with the Macbeth densitometer.

### [Moisture resistance test]

The thermal recording materials printed on in the thermal recordability test were stood for 24 hr in an environment of a test temperature 40°C and 90% RH, and the image densities of the recorded area and the non-image area were measured with the Macbeth densitometer.

**Table 1**

| | Developer | Sensitizer | | Color density | Heat resistance | Moisture resistance |
|---|---|---|---|---|---|---|
| Example 1* | 2,2'-ethylidenebis (4- methylphenol) | diphenyl- sulfone | recorded area | 1.20 | 0.97 | 0.91 |
| | | | non-image area | 0.09 | 0.10 | 0.07 |
| Example 2* | 2,2'-ethylidenebis(4- methylphenol) | 2-benzyloxy- naphthalene | recorded area | 1.27 | 0.84 | 0.89 |
| | | | non-image area | 0.08 | 0.09 | 0.06 |
| Example 3 | Condensed composition containing 2,2'-ethylidenebis (4-methylphenol) by 80% | diphenyl- sulfone | recorded area | 1.30 | 1.06 | 1.09 |
| | | | non-image area | 0.11 | 0.13 | 0.11 |
| Example 4 | Condensed composition containing 2,2'-ethylidenebis (4-methylphenol) by 80% | 1,2-bis- (phenoxy- methyl)- benzene | recorded area | 1.40 | 1.02 | 1.10 |
| | | | non-image area | 0.11 | 0.12 | 0.11 |
| Example 5 | Condensation composition containing 2,2'-ethylidenebis(4-t- butylphenol) by 70% | diphenyl- sulfone | recorded area | 1.29 | 1.12 | 1.11 |
| | | | non-image area | 0.09 | 0.11 | 0.13 |
| Example 6 | Condensed composition containing 2,2'-ethylidenebis(4-t- butylphenol) by 70% | ethylene glycol diphenyl ether | recorded area | 1.32 | 1.14 | 1.10 |
| | | | non-image area | 0.08 | 0.11 | 0.13 |
| Example 7* | 2,2'-ethylidenebis(4- cumylphenol) | diphenyl- sulfone | recorded area | 1.13 | 1.01 | 0.97 |
| | | | non-image area | 0.11 | 0.12 | 0.11 |
| Example 8* | 2,2'-ethylidenebis (4- cumylphenol) | 1,2-bis(3- methyl- phenoxy)- ethane | recorded area | 1.32 | 0.95 | 0.96 |
| | | | non-image area | 0.11 | 0.13 | 0.10 |
| Example 9* | 2,2'-butylidenebis(4- methylphenol) | diphenyl- sulfone | recorded area | 1.26 | 0.86 | 1.16 |
| | | | non-image area | 0.06 | 0.07 | 0.05 |
| Example 10* | 2,2'-butylidenebis(4- methylphenol) | 4-biphenyl- p-tolyl ether | recorded area | 1.32 | 0.79 | 1.19 |
| | | | non-image area | 0.06 | 0.07 | 0.05 |
| Example 11* | 2,2'-butylidenebis (4-t- butylphenol) | diphenyl- sulfone | recorded area | 1.32 | 1.04 | 1.03 |
| | | | non-image area | 0.06 | 0.07 | 0.05 |
| Example 12* | 2,2'-butylidenebis(4-t- butylphenol) | 4-biphenyl- p-tolyl ether | recorded area | 1.40 | 1.11 | 0.91 |
| | | | non-image area | 0.05 | 0.05 | 0.07 |

| | | | | | | |
|---|---|---|---|---|---|---|
| * Comparative, not according to the invention | | | | | | |

**Table 2**

| | Developer | Sensitizer | | Color density | Heat resistance | Moisture resistance |
|---|---|---|---|---|---|---|
| Comparative Example 1 | phenol novolac resin | none | recorded area | 0.37 | 0.20 | 0.15 |
| | | | non-image area | 0.08 | 0.08 | 0.11 |
| Comparative Example 2 | phenol novolac resin | diphenyl- sulfone | recorded area | 0.58 | 0.41 | 0.77 |
| | | | non-image area | 0.08 | 0.08 | 0.12 |

As is clear from Tables 1 and 2, the thermal recording materials of Examples 3 to 6 prepared using a developer comprising the condensed composition of formula (1) consisting of a condensate having two cores and, further, at least one kind of condensates having 3 to 5 cores, along with a sensitizer, are superior in preservation stability (heat resistance, moisture resistance), while maintaining high dynamic color density, as compared to the thermal recording materials of Comparative Examples 1 and 2 which used phenol novolac resin for the developer. In the Examples, moreover, thermal recording materials (Examples 3-6) containing a condensed composition as a developer could afford higher color density, and more superior preservation stability of recorded images.

### Industrial Applicability

According to the present invention, extremely useful thermal recording materials affording high color density of images and having superior preservation stability (particularly heat resistance, moisture resistance) of recorded images and the non-image area can be provided.

## Claims

1. A developer for a thermal recording material comprising a condensed composition of condensates represented by the formula (1): 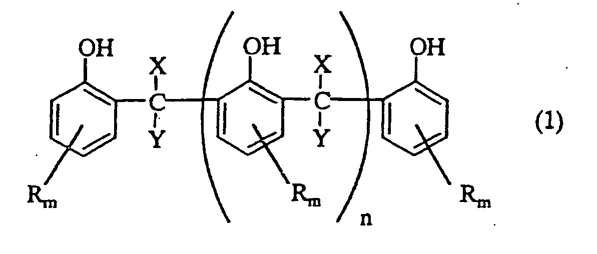 wherein
R is a hydrogen atom, a hydroxyl group, a lower alkyl group having 1 to 5 carbon atoms, an alkoxyl group having 1 to 5 carbon atoms, an aryl group or an aralkyl group,
R in the number of m
may be the same or different and m is an integer of 0-3,
X and Y are each a hydrogen atom, an alkyl group or an aryl group, provided that at least one of X and Y is an alkyl group or an aryl group, and
n is an integer of 0-3,
**characterized in that** the condensed composition consists of at least one kind of condensates of the formula (1) wherein n = 1-3 (3-5 core condensate), and 40-98% of the condensate of formula (1) having two cores wherein n = 0.

2. The developer for the thermal recording material of claim 1, wherein the condensate has a hydroxyl group, a lower alkyl group having 1 to 5 carbon atoms, an alkoxyl group having 1 to 5 carbon atoms, an aryl group or an aralkyl group at the p-position of a hydroxyl group of a phenol group.

3. A thermal recording material comprising a heat-coloring layer which comprises the developer of claim 1 or 2 and a basic dye.

4. The thermal recording material of claim 3 wherein the heat-coloring layer further comprises one or more kinds of sensitizers.

## Patentansprüche

1. Entwickler für ein wärmeempfindliches Aufzeichnungsmaterial, umfassend eine kondensierte Zusammensetzung von Kondensaten, die durch die Formel (1) 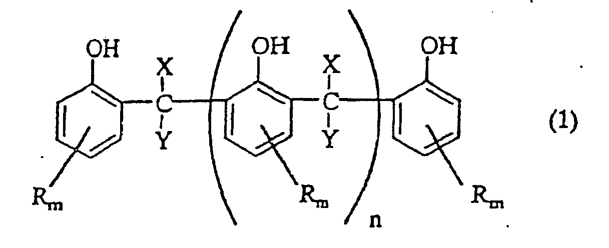 veranschaulicht sind, wobei
R ein Wasserstoffatom, eine Hydroxylgruppe, eine Niederalkylgruppe mit 1 bis 5 Kohlenstoffatomen, eine Alkoxygruppe mit 1 bis 5 Kohlenstoffatomen, eine Arylgruppe oder eine Aralkylgruppe ist,
R mit der Anzahl m
gleich oder verschieden sein kann und m eine ganze Zahl von 0-3 ist,
X und Y jeweils ein Wasserstoffatom, eine Alkylgruppe oder eine Aryl-gruppe sind, mit der Maßgabe, dass wenigstens einer der Reste X und Y eine Alkylgruppe oder eine Arylgruppe ist, und
n eine ganze Zahl von 0 - 3 ist,
**dadurch gekennzeichnet, dass** die kondensierte Zusammensetzung aus wenigstens einer Art von Kondensaten der Formel (1) besteht, wobei n = 1 - 3 (Kondensat mit 3-5 Kernen), und 40 - 98 % des Kondensats der Formel (1) zwei Kerne haben, wobei n = 0.

2. Entwickler für das wärmeempfindliche Aufzeichnungsmaterial nach Anspruch 1, wobei das Kondensat eine Hydroxylgruppe, eine Niederalkylgruppe mit 1 bis 5 Kohlenstoffatomen, eine Alkoxylgruppe mit 1 bis 5 Kohlenstoffatomen, eine Arylgruppe oder eine Aralkylgruppe an der p-Position der Hydroxylgruppe einer Phenolgruppe hat.

3. Wärmeempfindliches Aufzeichnungsmaterial, umfassend eine sich in der Wärme verfärbende Schicht, die den Entwickler nach Anspruch 1 oder 2 und einen basischen Farbstoff umfasst.

4. Wärmeempfindliches Aufzeichnungsmaterial nach Anspruch 3, wobei die sich in der Wärme verfärbende Schicht weiterhin ein oder mehrere Arten von Sensibilisatoren umfasst.

## Revendications

1. Révélateur pour matériau d'enregistrement thermique comprenant une composition condensée de condensats représentés par la formule (1) : 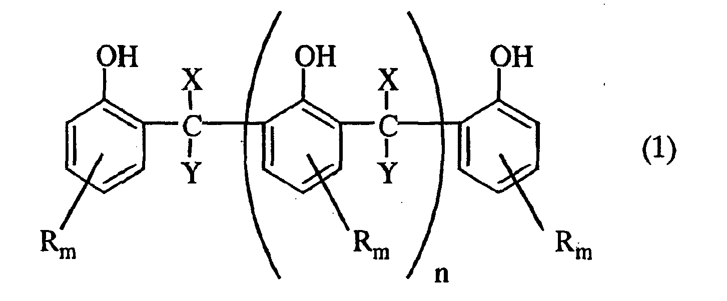 dans laquelle
R est un atome d'hydrogène, un groupe hydroxyle, un groupe alkyle inférieur ayant de 1 à 5 atomes de carbone, un groupe alcoxyle ayant de 1 à 5 atomes de carbone, un groupe aryle ou un groupe aralkyle,
R en nombres m peuvent être identiques ou différents, et m est un nombre entier de 0 à 3,
X et Y sont chacun un atome d'hydrogène, un groupe alkyle ou un groupe aryle, à condition qu'au moins un de X et Y soit un groupe alkyle ou un groupe aryle, et
n est un nombre entier de 0 à 3,
**caractérisé en ce que** la composition condensée est constituée d'au moins un type de condensats de formule (1) où n = 1 à 3 (3 à 5 noyaux de condensat), et de 40 à 98 % du condensat de formule (1) ayant deux noyaux où n = 0.

2. Révélateur pour matériau d'enregistrement thermique selon la revendication 1, dans lequel le condensat a un groupe hydroxyle, un groupe alkyle inférieur ayant de 1 à 5 atomes de carbone, un groupe alcoxyle ayant de 1 à 5 atomes de carbone, un groupe aryle ou un groupe aralkyle en position p d'un groupe hydroxyle d'un groupe phénol.

3. Matériau d'enregistrement thermique comprenant une couche se colorant sous l'effet de la chaleur qui comprend le révélateur selon la revendication 1 ou 2 et un colorant basique.

4. Matériau d'enregistrement thermique selon la revendication 3, dans lequel la couche se colorant sous l'effet la chaleur comprend en outre un ou plusieurs types de sensibilisateurs.
